# EUROPÄISCHE PATENTSCHRIFT

(11) **EP 3 527 576 B1**
(45) Veröffentlichungstag und Bekanntmachung des Hinweises auf die Patenterteilung: **18.11.2020**
(21) Anmeldenummer: 18156675.3
(22) Anmeldetag: 14.02.2018
(51) Int. Cl.: C07F 9/58, C07C 67/38, C07F 15/00

(54) **PROPYL-VERBRÜCKTE DIPHOSPHINLIGANDEN FÜR DIE ALKOXYCARBONYLIERUNG**
PROPYL-BRIDGED DIPHOSPHINE LIGANDS FOR ALKOXYCARBONYLATION
LIGANDS DE DISPHOSPHINE RÉCULÉS PAR PROPYL POUR L'ALKOXY-CARBONYLATION

(43) Veröffentlichungstag der Anmeldung: 21.08.2019
(73) Patentinhaber: Evonik Operations GmbH, 45128 Essen (DE)
(72) Erfinder: LIU, Jiawang, Shisanliquiao Town 464137 City Henan (CN); DONG, Kaiwu, 236800 Bo Zhou (CN); FRANKE, Robert, 45772 Marl (DE); NEUMANN, Helfried, 18055 Rostock (DE); JACKSTELL, Ralf, 10106 Rostock (DE); BELLER, Matthias, 18211 Ostseebad Nienhagen (DE)
(74) Vertreter: Evonik Patent Association

(56) Entgegenhaltungen:
- US-A- 5 175 244
- US-A1- 2017 022 138

## Beschreibung

Die Erfindung betrifft propyl-verbrückte Diphosphinverbindungen, Metallkomplexe dieser Verbindungen und deren Verwendung für die Alkoxycarbonylierung.

Die Alkoxycarbonylierung ethylenisch ungesättigter Verbindungen ist ein Prozess mit steigender Bedeutung. Unter einer Alkoxycarbonylierung versteht man die Umsetzung ethylenisch ungesättigter Verbindungen (Olefinen) mit Kohlenmonoxid und Alkoholen in Gegenwart eines Metall-Ligand-Komplexes zu den entsprechenden Estern. Üblicherweise wird als Metall Palladium eingesetzt. Das folgende Schema zeigt die allgemeine Reaktionsgleichung einer Alkoxycarbonylierung:

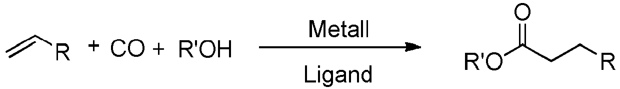

In EP 3 121 180 A2 wird ein Verfahren zur Alkoxycarbonylierung und die hierzu verwendeten Liganden beschrieben. Bei diesem Verfahren kommen butyl-verbrückte Diphosphinverbindungen zum Einsatz.

Die vorliegende Erfindung hat die Aufgabe, neue Liganden für die Alkoxycarbonylierung bereitzustellen, mit denen sich gute Ausbeuten an Estern erzielen lassen.

Diese Aufgabe wird gelöst durch Verbindungen nach Anspruch 1.

Verbindung gemäß der Formel (1) 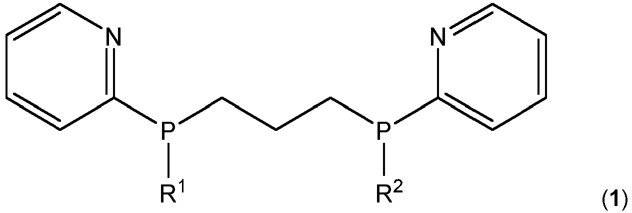
wobei R¹ für ^{t}Bu steht und
R² für -(C₁-C₁₂)-Alkyl steht.

In einer Ausführungsform stehen R¹ und R² für den gleichen Rest.

In einer Ausführungsform steht R² für ^{t}Bu.

In einer Ausführungsform weist die Verbindung die Struktur (L1) auf:

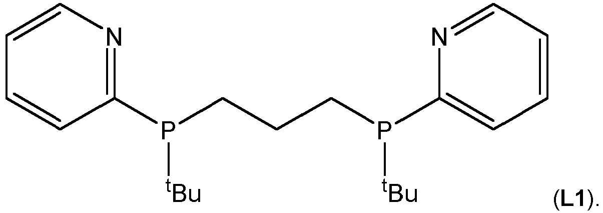

Neben den zuvor beschriebenen Verbindungen wird auch ein Komplex beanspruch, welcher eine der zuvor beschrieben Verbindungen und Pd umfasst.

Komplex umfassend Pd und eine zuvor beschriebene Verbindung.

Neben dem Komplex und der Verbindung, wird auch ein Verfahren beansprucht, in welchem diese zum Einsatz kommen.

Verfahren umfassend die Verfahrensschritte:
a) Vorlegen einer ethylenisch ungesättigten Verbindung;
b) Zugabe einer zuvor beschriebenen Verbindung und einer Verbindung, welche Pd umfasst,
   oder Zugabe eines zuvor beschriebenen Komplexes;
c) Zugabe eines Alkohols;
d) Zuführen von CO;
e) Erwärmen des Reaktionsgemisches, wobei die ethylenisch ungesättigte Verbindung zu einem Ester umgesetzt wird.

Hierbei können die Verfahrensschritte a), b), c) und d) in beliebiger Reihenfolge erfolgen. Üblicherweise erfolgt die Zugabe von CO jedoch, nachdem die Reaktionspartner in den Schritten a) bis c) vorgelegt wurden. Die Schritte d) und e) können gleichzeitig oder nacheinander erfolgen. Darüber hinaus kann CO auch in mehreren Schritten zugeführt werden, so dass beispielsweise zunächst ein Teil des CO zugeführt, dann erwärmt und anschließend ein weiterer Teil CO zugeführt wird.

In einer Variante des Verfahrens ist die ethylenisch ungesättigte Verbindung ausgewählt aus: Ethen, Propen, 1-Buten, cis-2-Buten, trans-2-Buten, iso-Buten, 1,3-Butadien, 1-Penten, cis-2-Penten trans-2-Penten, 2-Methyl-1-buten, 3-Methyl-1-buten, 2-Methyl-2-buten, Hexen, Tetramethylethylen (2,3-Dimethyl-2-buten), Hepten, 1-Octen, 2-Octen, di-n-Buten, oder Mischungen davon.

In einer Variante des Verfahrens ist die ethylenisch ungesättigte Verbindung Tetramethylethylen.

In einer Variante des Verfahrens ist die Verbindung in Verfahrensschritt b), welche Pd umfasst, ausgewählt aus:
PdCl₂, PdBr₂, Pd(acac)₂, Pd(dba)₂ (dba = Dibenzylidenaceton), PdCl₂(CH₃CN)₂.

In einer Variante des Verfahrens ist die Verbindung in Verfahrensschritt b), welche Pd umfasst, Pd(acac)₂.

In einer Variante des Verfahrens ist der Alkohol in Verfahrensschritt c) ausgewählt aus: Methanol, Ethanol, 1-Propanol, 1-Butanol, 1-Pentanol, 1-Hexanol, 2-Propanol, tert-Butanol, 3-Pentanol, Cyclohexanol, Phenol, oder Mischungen davon.

In einer Variante des Verfahrens ist der Alkohol in Verfahrensschritt c) Methanol.

In einer Variante des Verfahrens wird das Reaktionsgemisch im Verfahrensschritt e) auf eine Temperatur im Bereich von 80 °C bis 160 °C erwärmt.

In einer Variante des Verfahrens wird das Reaktionsgemisch im Verfahrensschritt e) auf eine Temperatur im Bereich von 100 °C bis 140 °C erwärmt.

In einer Variante des Verfahrens erfolgt das Zuführen von CO im Verfahrensschritt d) so, dass die Reaktion bei einem CO-Druck im Bereich von 2000 kPa (20 bar) bis 5000 kPa (50 bar) verläuft.

In einer Variante des Verfahrens erfolgt das Zuführen von CO im Verfahrensschritt d) so, dass die Reaktion bei einem CO-Druck im Bereich von 3000 kPa (30 bar) bis 5000 kPa (50 bar) verläuft.

In einer Variante des Verfahrens umfasst das Verfahren den zusätzlichen Verfahrensschritt f): f) Zugabe von p-Toluolsulfonsäure.

Im Folgenden wird die Erfindung anhand von Ausführungsbeispielen näher erläutert.

### Allgemeine Arbeitsvorschriften

Alle nachfolgenden Präparationen wurden mit Standard-Schlenk-Technik unter Schutzgas durchgeführt. Die Lösungsmittel wurden vor Gebrauch über geeigneten Trocknungsmitteln getrocknet (Purification of Laboratory Chemicals, W. L. F. Armarego (Autor), Christina Chai (Autor), Butterworth Heinemann (Elsevier), 6. Auflage, Oxford 2009).

Phosphortrichlorid (Aldrich) wurde vor dem Einsatz unter Argon destilliert. Alle präparativen Arbeiten erfolgten in ausgeheizten Gefäßen. Die Charakterisierung der Produkte erfolgte mittels NMR-Spektroskopie. Chemische Verschiebungen (δ) werden in ppm angegeben. Die Referenzierung der ³¹P-NMR-Signale erfolgte gemäß: SR_{31P} = SR_{1H} * (BF_{31P} / BF_{1H}) = SR_{1H} * 0,4048. (Robin K. Harris, Edwin D. Becker, Sonia M. Cabral de Menezes, Robin Goodfellow, und Pierre Granger, Pure Appl. Chem., 2001, 73, 1795-1818; Robin K. Harris, Edwin D. Becker, Sonia M. Cabral de Menezes, Pierre Granger, Roy E. Hoffman und Kurt W. Zilm, Pure Appl. Chem., 2008, 80, 59-84).

Die Aufnahme von Kernresonanzspektren erfolgte an Bruker Avance 300 bzw. Bruker Avance 400, die gaschromatografische Analyse an Agilent GC 7890A, die ESI-TOF Massenspektrometrie an Thermo Electron Finnigan MAT 95-XP und Agilent 6890 N/5973 Geräten.

### Darstellung von Chlor-2-pyridyl-tert-butylphosphin (Vorstufe A)

Der Grignard für die Synthese von Chlor-2-pyridyl-t-butylphosphin wird nach der "Knochelmethode" mit Isopropylmagnesiumchlorid dargestellt (Angew. Chem. 2004, 43, 2222-2226). Die Aufarbeitung erfolgt nach der Methode von Budzelaar (Organometallics 1990, 9, 1222-1227).

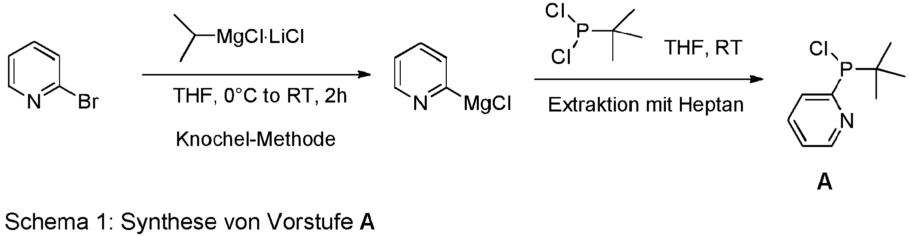

In einen 50 ml-Rundkolben mit Magnetrührer und Septum werden unter Argon 8,07 ml einer 1,3 M Isopropylmagnesium-chloridlösung (Knochel-Reagenz) gegeben und auf -15 °C gekühlt. Danach werden zügig 954 µ1 (10 mmol) 2-Bromopyridin zugetropft. Die Lösung wird sofort gelb. Man lässt auf-10 °C aufwärmen. Der Umsatz der Reaktion wird wie folgt bestimmt: man entnimmt ca. 100 µl Lösung und gibt sie in 1 ml einer gesättigten Ammoniumchloridlösung. Wenn die Lösung "sprudelt", dann hat sich noch nicht viel Grignard gebildet. Die wässrige Lösung wird mit einer Pipette Ether extrahiert und die organische Phase über Na₂SO₄ getrocknet. Von der etherischen Lösung wird ein GC aufgenommen. Wenn sich viel Pyridin gebildet hat im Vergleich zu 2-Bromopyridin, dann hat man hohe Umsätze. Bei -10 °C hat sich wenig umgesetzt. Nach Aufwärmen auf Raumtemperatur und 1-2 stündigen Rühren wird die Reaktionslösung braungelb. Ein GC-Test zeigt vollständigen Umsatz. Jetzt wird die Grignardlösung zu einer Lösung von 1,748 g (11 mmol) Dichloro-tert-butylphosphin in 10 ml THF, die vorher auf -15 °C gekühlt worden ist, mit einer Spritzenpumpe langsam zugetropft. Die Dichloro-tert-butylphosphinlösung wird gekühlt. Es entsteht anfangs eine klare gelbe Lösung, die dann trübe wird. Man lässt auf Raumtemperatur aufwärmen und über Nacht Rühren. Man entfernt das Lösungsmittel im Hochvakuum und erhält einen weißlichen Feststoff, der braune Stellen enthält. Der Feststoff wird mit 20 ml Heptan suspendiert und der Feststoff wird im Ultraschallbad zerkleinert. Nach dem Absetzen lassen des weißen Feststoffes wird die Lösung dekantiert. Der Vorgang wird zwei Mal mit je 10-20 ml Heptan wiederholt. Nach dem Einengen der Heptanlösung im Hochvakuum wird diese unter Vakuum destilliert. Bei 4,6 mbar, 120 °C Ölbad und 98 °C Übergangstemperatur kann das Produkt destilliert werden. Man erhält 1,08 g eines farblosen Öls. (50%).

### Analytische Daten:

¹H NMR (300 MHz, C₆D₆): δ 8,36 (m, 1H, Py), 7,67 (m, 1H, Py), 7,03-6,93 (m, 1H, Py), 6,55-6,46 (m, 1H, Py), 1,07 (d, J = 13,3 Hz, 9H, t-Bu).
¹³C NMR (75 MHz, C₆D₆): δ 162,9, 162,6, 148,8, 135,5, 125,8, 125,7, 122,8, 35,3, 34,8, 25,9 und 25,8.
³¹P NMR (121 MHz, C₆D₆) δ 97,9.
MS (EI) *m*:*z* (relative Intensität) 201 (M⁺,2), 147(32), 145 (100), 109 (17), 78 (8), 57,1 (17).

### Herstellung von Verbindung (L1)

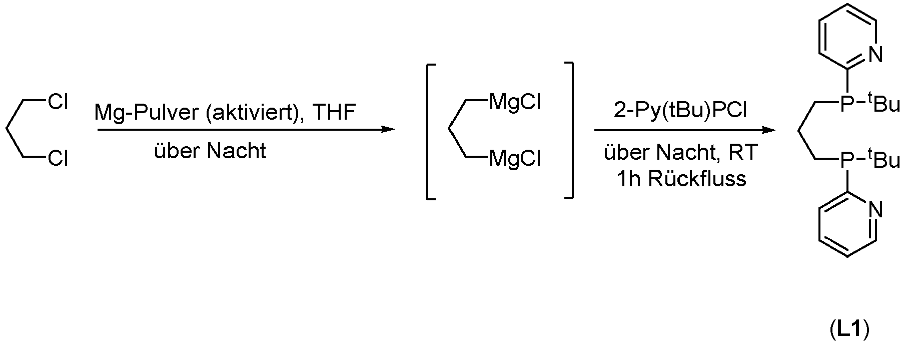

(analog zu Graham Eastham et al., US 6,335,471)

### Vergleichsliganden

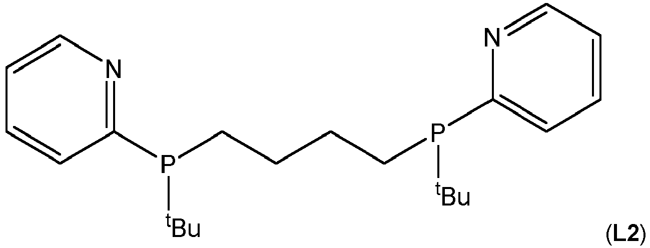

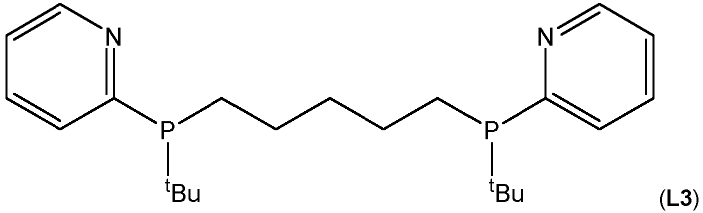

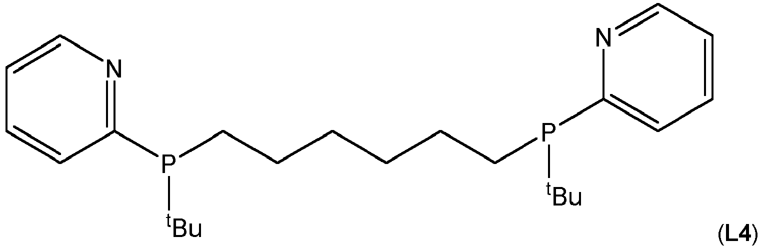

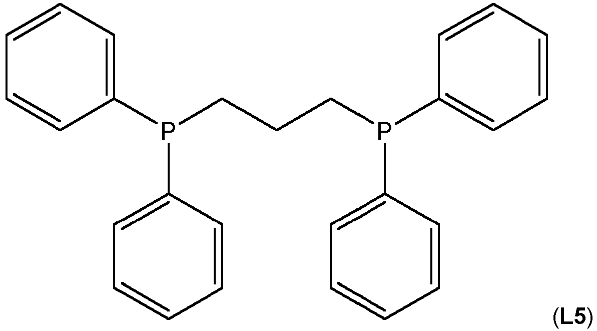

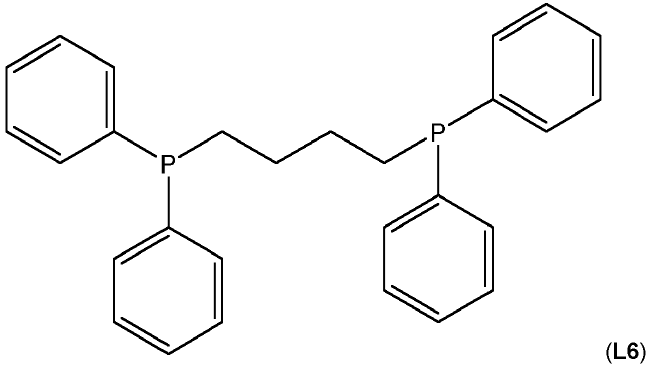

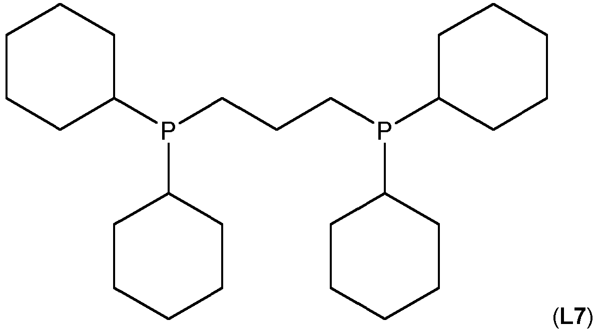

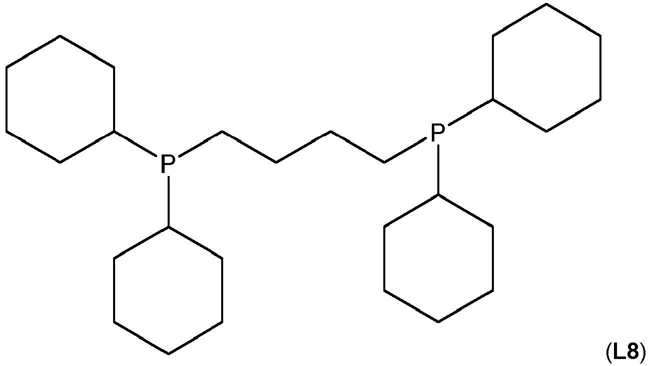

### Methoxycarbonylierung von Tetramethylethylen (1a)

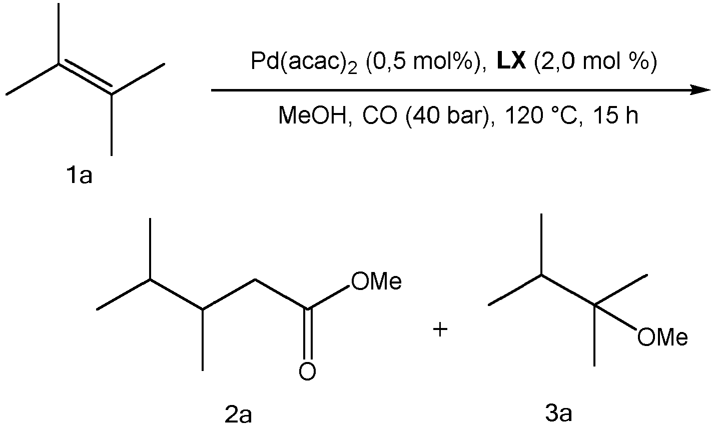

### Allgemeine Versuchsvorschrift für Autoklavenversuche in Glasvials:

Es wird ein 300ml Parrreaktor verwendet. Diesem angepasst ist ein selbstgefertigter Aluminiumblock entsprechender Dimension welcher zu Heizen mittels handelsüblicher Magnetrührer z.B. der Firma Heidolph geeignet ist. Für das Innere des Autoklaven wurde eine runde Metallplatte der Stärke von ca. 1,5 cm angefertigt die 6 Bohrungen enthält, die dem Außendurchmesser der Glasvials entsprechen. Diesen Glasvials angepasst werden sie mit kleinen Magnetrührern bestückt. Diese Glasvials werden mit Schraubkappen und geeigneten Septen versehen und mit einer in der Glasbläserei angefertigten Spezialapparatur unter Argon mit den entsprechenden Reaktanden, Lösungsmittel und Katalysatoren und Additiven befüllt. Hierzu werden 6 Gefäße gleichzeitig befüllt, dies ermöglicht die Durchführung von 6 Reaktionen bei gleicher Temperatur und gleichem Druck in einem Experiment. Dann werden diese Glasgefäße mit Schraubkappen und Septen geschlossen und es wird jeweils ein kleine Spritzenkanüle geeigneter Größe durch die Septen gestochen. Dies ermöglicht später in der Reaktion den Gasaustausch. Diese Vials werden nun in der Metallplatte platziert und diese wird unter Argon in den Autoklaven überführt. Der Autoklav wird mit CO gespült und bei Raumtemperatur mit dem vorgesehenen CO-Druck befüllt. Dann wird mittels dem Magnetrührer unter Magnetrührung auf Reaktionstemperatur erhitzt und die Reaktion die entsprechende Zeit durchgeführt. Anschließend wird auf Raumtemperatur heruntergekühlt und der Druck langsam abgelassen. Anschließend wird der Autoklav mit Stickstoff gespült. Die Vials werden dem Autoklaven entnommen und mit einer definierten Menge eines geeigneten Standards versetzt. Es erfolgt eine GC Analyse mit deren Ergebnissen Ausbeuten und Selektivitäten bestimmt wurden.

### Methoxycarbonylierung

Ein 4 mL-Fläschchen wurde mit Tetramethylethylen (1a) (1,0 mol) befüllt und ein Magnetrührstab hinzugegeben. Anschließend wurden hinzugegeben: Pd(acac)₂ (1,52 mg, 0,5 mol%), L1 (2,0 mol%), PTSA*H₂O (16,0 mg, 8,0 mol%), MeOH (2,0 ml). Das Fläschchen wurde auf einen Probenhalter gesetzt, der wiederum unter Argon-Atmosphäre in einen 300 ml-Parr-Autoklaven eingesetzt wurde. Nach dreimaligem Spülen des Autoklaven mit Stickstoff wurde der CO-Druck auf 4000 kPa (40 bar) eingestellt. Die Reaktion lief bei 120 °C 15 Stunden. Nach Abschluss der Reaktion wurde der Autoklav auf Raumtemperatur abgekühlt und vorsichtig entspannt. Ausbeute und Regioselektivität wurden mittels GC bestimmt.

Der oben beschriebene Versuch wurde mit den Vergleichsliganden (L2) bis (L8) wiederholt. Alle anderen Parameter wurden beibehalten. Die Ergebnisse der Versuchsreihe sind in der nachfolgenden Tabelle zusammengestellt:

**Tabelle:**

| Ligand (**LX**) | Umsatz [%] | Ausbeute **2a** [%] | Ausbeute **3a** [%] |
|---|---|---|---|
| **L1*** | > 99 | 99 | 0 |
| **L2** | 92 | 84 | 7 |
| **L3** | 49 | 4 | 41 |
| **L4** | 52 | 2 | 44 |
| **L5** | 48 | 2 | 43 |
| **L6** | 63 | 22 | 31 |
| **L7** | 54 | 0 | 45 |
| **L8** | 48 | 0 | 45 |

| | | | |
|---|---|---|---|
| * erfindungsgemäße Verbindung | | | |

Wie die oben gezeigten Ergebnisse zeigen, wird die Aufgabe durch die erfindungsgemäße Verbindung gelöst.

## Patentansprüche

1. Verbindung gemäß der Formel (1) 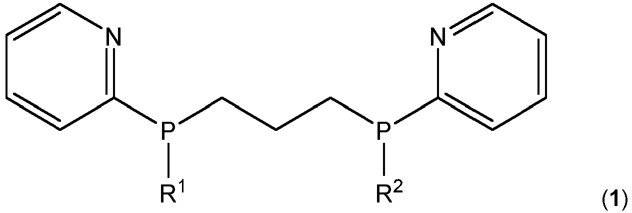
wobei R¹ für ^{t}Bu steht und
R² für -(C₁-C₁₂)-Alkyl steht.

2. Verbindung nach Anspruch 1,
wobei R¹ und R² für den gleichen Rest stehen.

3. Verbindung nach einem der Ansprüche 1 oder 2,
wobei R² für ^{t}Bu steht.

4. Verbindung nach einem der Ansprüche 1 bis 3,
wobei diese die Struktur (L1) aufweist:

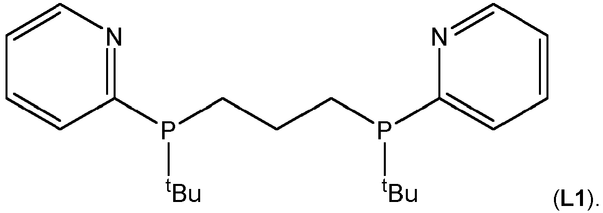

5. Komplex umfassend Pd und eine Verbindung nach einem der Ansprüche 1 bis 4.

6. Verfahren umfassend die Verfahrensschritte:
a) Vorlegen einer ethylenisch ungesättigten Verbindung;
b) Zugabe einer Verbindung nach einem der Ansprüche 1 bis 4 und einer Verbindung, welche Pd umfasst,
oder Zugabe eines Komplexes nach Anspruch 5;
c) Zugabe eines Alkohols;
d) Zuführen von CO;
e) Erwärmen des Reaktionsgemisches, wobei die ethylenisch ungesättigte Verbindung zu einem Ester umgesetzt wird.

7. Verfahren nach Anspruch 6,
wobei die ethylenisch ungesättigte Verbindung ausgewählt ist aus:
Ethen, Propen, 1-Buten, *cis*-2-Buten, *trans*-2-Buten, iso-Buten, 1,3-Butadien, 1-Penten, *cis-*2-Penten *trans*-2-Penten, 2-Methyl-1-buten, 3-Methyl-1-buten, 2-Methyl-2-buten, Hexen, Tetramethylethylen (2,3-Dimethyl-2-buten), Hepten, 1-Octen, 2-Octen, di-n-Buten, oder Mischungen davon.

8. Verfahren nach einem der Ansprüche 6 oder 7,
wobei die Verbindung in Verfahrensschritt b), welche Pd umfasst, ausgewählt ist aus: PdCl₂, PdBr₂, Pd(acac)₂, Pd(dba)₂ (dba = Dibenzylidenaceton), PdCl₂(CH₃CN)₂.

9. Verfahren nach einem der Ansprüche 6 bis 8,
wobei der Alkohol in Verfahrensschritt c) ausgewählt ist aus:
Methanol, Ethanol, 1-Propanol, 1-Butanol, 1-Pentanol, 1-Hexanol, 2-Propanol, *tert*-Butanol, 3-Pentanol, Cyclohexanol, Phenol, oder Mischungen davon.

10. Verfahren nach einem der Ansprüche 6 bis 9,
wobei das Reaktionsgemisch im Verfahrensschritt e) auf eine Temperatur im Bereich von 80 °C bis 160 °C erwärmt wird.

11. Verfahren nach einem der Ansprüche 6 bis 10,
wobei das Zuführen von CO im Verfahrensschritt d) so erfolgt, dass die Reaktion bei einem CO-Druck im Bereich von 2000 kPa50 (20 bar) bis 5000 kPa (50 bar) verläuft.

12. Verfahren nach einem der Ansprüche 6 bis 11,
wobei das Verfahren den zusätzlichen Verfahrensschritt f) umfasst:
f) Zugabe von p-Toluolsulfonsäure.

## Claims

1. Compound of the formula (1) 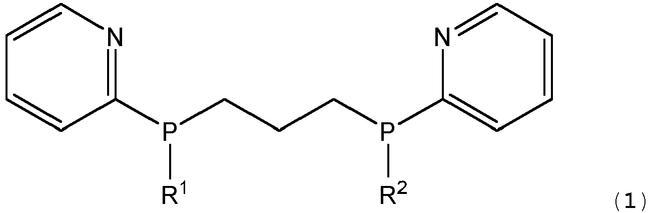
where R¹ is ^{t}Bu and
R² is -(C₁-C₁₂)-alkyl.

2. Compound according to Claim 1,
where R¹ and R² are the same radical.

3. Compound according to either of Claims 1 and 2,
where R¹ is ^{t}B_{u.}

4. Compound according to any of Claims 1 to 3,
wherein it has the structure (**L1**):

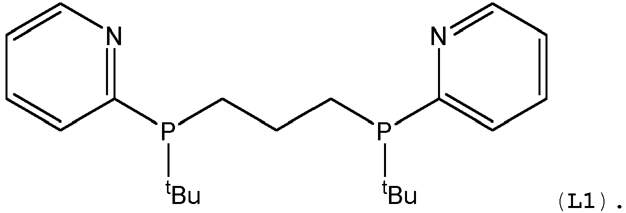

5. Complex comprising Pd and a compound according to any of Claims 1 to 4.

6. Process comprising the process steps of:
a) initially charging an ethylenically unsaturated compound;
b) adding a compound according to any of Claims 1 to 4 and a compound comprising Pd,
or adding a complex according to Claim 5;
c) adding an alcohol;
d) feeding in CO;
e) heating the reaction mixture, with conversion of the ethylenically unsaturated compound to an ester.

7. Process according to Claim 6,
wherein the ethylenically unsaturated compound is selected from:
ethene, propene, 1-butene, cis-2-butene, trans-2-butene, isobutene, 1,3-butadiene, 1-pentene, cis-2-pentene, trans-2-pentene, 2-methyl-1-butene, 3-methyl-1-butene, 2-methyl-2-butene, hexene, tetramethylethylene (2,3-dimethyl-2-butene), heptene, 1-octene, 2-octene, di-n-butene, or mixtures thereof.

8. Process according to either of Claims 6 and 7,
wherein the compound in process step b), comprising Pd, is selected from:
PdCl₂, PdBr₂, Pd(acac)₂, Pd(dba)₂ (dba = dibenzylideneacetone), PdCl₂ (CH₃CN)₂.

9. Process according to any of Claims 6 to 8,
wherein the alcohol in process step c) is selected from:
methanol, ethanol, 1-propanol, 1-butanol, 1-pentanol, 1-hexanol, 2-propanol, tert-butanol, 3-pentanol, cyclohexanol, phenol, or mixtures thereof.

10. Process according to any of Claims 6 to 9,
wherein the reaction mixture is heated in process step e) to a temperature in the range from 80°C to 160°C.

11. Process according to any of Claims 6 to 10,
wherein CO is fed in in process step d) such that the reaction proceeds at a CO pressure in the range from 2000 kPa50 (20 bar) to 5000 kPa (50 bar).

12. Process according to any of Claims 6 to 11,
wherein the process comprises the additional process step f) :
f) adding p-toluenesulfonic acid.

## Revendications

1. Composé selon la formule (1) 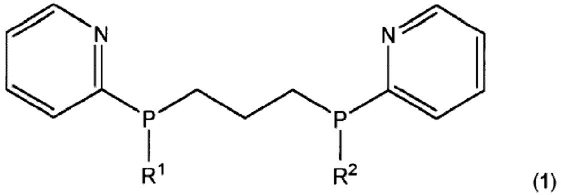
dans laquelle R¹ représente un radical ^{t}Bu et
R² représente un radical -alkyle en C₁-C₁₂.

2. Composé selon la revendication 1,
dans lequel R¹ et R² représentent le même radical.

3. Composé selon l'une quelconque des revendications 1 et 2,
dans lequel R² représente un radical ^{t}Bu.

4. Composé selon l'une quelconque des revendications 1 à 3,
dans lequel ce dernier présente la structure (**L1**) :

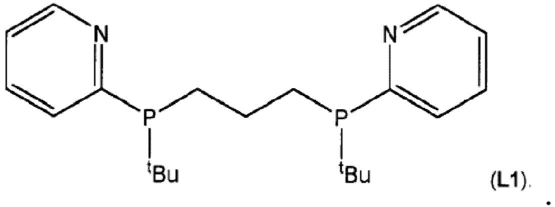

5. Complexe comprenant du Pd et un composé selon l'une quelconque des revendications 1 à 4.

6. Procédé comprenant les étapes de processus suivantes :
a) disposition d'un composé à insaturation éthylénique ;
b) addition d'un composé selon l'une quelconque des revendications 1 à 4 et d'un composé qui comprend du Pd,
ou addition d'un complexe selon la revendication 5 ;
c) addition d'un alcool ;
d) introduction de CO ;
e) chauffage du mélange réactionnel, lors duquel le composé à insaturation éthylénique est converti en un ester.

7. Procédé selon la revendication 6,
dans lequel le composé à insaturation éthylénique est choisi parmi :
l'éthène, le propène, le 1-butène, le cis-2-butène, le *trans*-2-butène, l'isobutène, le 1,3-butadiène, le 1-pentène, le cis-2-pentène, le *trans-*2-pentène, le 2-méthyl-1-butène, le 3-méthyl-1-butène, le 2-méthyl-2-butène, l'hexène, le tétraméthyléthylène (2,3-diméthyl-2-butène), l'heptène, le 1-octène, le 2-octène, le di-n-butène, ou des mélanges de ceux-ci.

8. Procédé selon l'une quelconque des revendications 6 et 7,
dans lequel le composé dans l'étape b) du processus, qui comprend du Pd, est choisi parmi PdCl₂, PdBr₂, Pd(acac)₂, Pd(dba)₂ (dba = dibenzylacétone), PdCl₂(CH₃CN)₂.

9. Procédé selon l'une quelconque des revendications 6 à 8,
dans lequel l'alcool dans l'étape c) du processus est choisi parmi :
le méthanol, l'éthanol, le 1-propanol, le 1-butanol, le 1-pentanol, le 1-hexanol, le 2-propanol, le tert-butanol, le 3-pentanol, le cyclohexanol, le phénol, ou des mélanges de ceux-ci.

10. Procédé selon l'une quelconque des revendications 6 à 9,
dans lequel on chauffe le mélange réactionnel dans l'étape e) du processus à une température dans la plage de 80 °C à 160 °C.

11. Procédé selon l'une quelconque des revendications 6 à 10,
dans lequel l'introduction de CO dans l'étape d) du processus s'effectue de telle façon que la réaction se déroule sous une pression de CO dans la plage de 2 000 kPa50 (20 bars) à 5 000 kPa (50 bars).

12. Procédé selon l'une quelconque des revendications 6 à 11,
le procédé comprenant l'étape de processus f) supplémentaire :
f) addition d'acide p-toluènesulfonique.
